# EUROPEAN PATENT SPECIFICATION

(11) **EP 2 971 324 B1**
(45) Date of publication and mention of the grant of the patent: **31.01.2018**
(21) Application number: 14770433.2
(22) Date of filing: 13.03.2014
(51) Int. Cl.: G01N 33/569, G01N 33/58, D06M 10/08, D06M 10/00, D06M 15/643, D06M 13/07, D06M 16/00, D06M 13/352, D06M 13/513

(54) **MICROWAVE INITIATION FOR DEPOSITION OF POROUS ORGANOSILICATE MATERIALS ON FABRICS**
MIKROWELLENINITIATION ZUR ABSCHEIDUNG VON PORÖSEN ORGANOSILIKATMATERIALIEN AUF GEWEBEN
DÉCLENCHEMENT DE MICRO-ONDES POUR PERMETTRE UN DÉPÔT DE MATÉRIAUX ORGANOSILICÉS POREUX SUR DES TISSUS

(30) Priority: 14.03.2013 US 201361784260 P
(43) Date of publication of application: 20.01.2016
(73) Proprietor: The Government of the United States of America as represented by the Secretary of the Navy, Arlington, Virginia 22203 (US)
(72) Inventor: WHITE, Brandy, J, Washington, DC 20002 (US); MELDE, Brian, Alexandria, VA 22304 (US)
(74) Representative: Dunleavy, Kevin James
(86) International application number: PCT/US2014/026398
(87) International publication number: WO 2014/151756

(56) References cited:
- WO-A1-98/39497
- WO-A1-2010/087887
- CN-A- 101 804 327
- KR-A- 20110 024 830
- US-A- 5 085 938
- US-B2- 8 062 414
- RYAN A HAYN ET AL: "Preparation of highly hydrophobic and oleophobic textile surfaces using microwave-promoted silane coupling", JOURNAL OF MATERIALS SCIENCE, KLUWER ACADEMIC PUBLISHERS, BO, vol. 46, no. 8, 4 December 2010 (2010-12-04), pages 2503-2509, XP019876895, ISSN: 1573-4803, DOI: 10.1007/S10853-010-5100-5
- DATABASE WPI Week 200364 Thomson Scientific, London, GB; AN 2003-673812 XP002761681, -& JP 2003 082557 A (TORAY IND INC) 19 March 2003 (2003-03-19)
- RINGOT C ET AL: "Porphyrin-grafted cellulose fabric: New photobactericidal material obtained by ''Click-Chemistry'' reaction", MATERIALS LETTERS, NORTH HOLLAND PUBLISHING COMPANY. AMSTERDAM, NL, vol. 63, no. 21, 31 August 2009 (2009-08-31), pages 1889-1891, XP026250828, ISSN: 0167-577X, DOI: 10.1016/J.MATLET.2009.06.009 [retrieved on 2009-06-10]
- DATABASE WPI Week 200915 Thomson Scientific, London, GB; AN 2009-E62816 XP002761682, -& JP 2009 030200 A (DOKURITSU GYOSEI HOJIN SANGYO GIJUTSU SO) 12 February 2009 (2009-02-12)
- BRANDY J. JOHNSON ET AL: "Fluorescent Silicate Materials for the Detection of Paraoxon", SENSORS, vol. 10, no. 3, 19 March 2010 (2010-03-19) , pages 2315-2331, XP055301301, CH ISSN: 1424-8220, DOI: 10.3390/s100302315
- BRANDY J JOHNSON ET AL: "Functionalized organosilicate materials for irritant gas removal", CHEMICAL ENGINEERING SCIENCE, OXFORD, GB, vol. 68, no. 1, 25 September 2011 (2011-09-25), pages 376-382, XP028107807, ISSN: 0009-2509, DOI: 10.1016/J.CES.2011.09.048 [retrieved on 2011-10-01]

## Description

### BACKGROUND

Traditional protective garments and shelters frequently utilize carbon materials such as activated carbon. These materials are typically reliant on non-specific adsorption, may provide little to no catalytic/reactive activity, and do not protect against the full range of threat agents. Furthermore, protective garments often provide little to no water transport across protective barriers, resulting in discomfort to the wearer and limiting the duration of their use. A need exists for fabrics addressing these short-comings.

JP 2003 082557A of TORAY IND INC discloses a metallic fiber nonwoven fabric-immobilized mesostructure. The mesostructured is immobilized with mesoporous silica or mesoporous silica containing at least one microparticle component selected from metallic microparticles and metal oxide microparticles. The non-woven fabric is used to filter and decompose chemical contaminants.

RINGOT C ET AL. "Porphyrin-grafted cellulose fabric: New photobactericidal material obtained by "Click-Chemistry" reaction", MATERIALS LETTERS, NORTH HOLLAND PUBLISHING COMPANY. AMSTERDAM, NL, discloses the grafting of porphyrin on cellulose fabric to produce a modified fabric. The porphyrin is grafted onto the fabric using "click chemistry"

JP 2009 030200A of NIPPON SEISEN CO LTD discloses a metallic fiber non-woven fabric-immobilized mesostructure produced by solidifying silicon alkoxide fabric by hydrolysis in the metal fiber non-woven fabric.

CN101804327 discloses a carbon dioxide absorbing material. The material is an organic amine-mesoporous molecular sieve-cotton fiber formed composite material. In the material, a larger space is maintained between cotton fibers, and thus effective carbon dioxide absorption can occur. The material is said to have high efficiency and high stability.

### BRIEF SUMMARY

In a first embodiment, a modified fabric includes a modified fabric modified by by wetting the fabric in a first solution comprising tetraethylorthosilicate (TEOS) to obtain a precursor fabric, irradiating the precursor fabric with microwave radiation to obtain a TEOS functionalized fabric, dip-coating the TEOS functionalized fabric in a dip solution comprising surfactant and organosilica precursor to obtain dipped fabric, and curing the dipped fabric to obtain the modified fabric.

In another embodiment, a method of treating fabric includes wetting a fabric in a first solution comprising tetraethylorthosilicate (TEOS) to obtain a precursor fabric, irradiating the precursor fabric with microwave radiation to obtain a TEOS functionalized fabric, dip-coating the TEOS functionalized fabric in a dip solution comprising surfactant and organosilica precursor to obtain dipped fabric, and curing the dipped fabric to obtain a modified fabric. The organosilica precursor comprises: (1) bis(trimethoxysilyl)ethane (BTE) and 1,4-bis(trimethoxysilylethyl)benzene (DEB); or (2) mesitylene and BTE.

### BRIEF DESCRIPTION OF THE DRAWINGS

FIGs. 1A - IF show scanning electron microscopy (SEM) images of fabrics before and after tetraethylorthosilicate (TEOS) functionalization: unbleached cotton (A), ACU (B), multi-cam (C) and of TEOS functionalized fabrics: 1 cycle (D), 3 cycles (E), 10 cycles (F).
FIGs. 2A and 2B show nitrogen adsorption (A) and pore size distribution (B) for fabric modification using bis(trimethoxysilylethyl)benzene (DEB) and bis(trimethoxysilyl)ethane (BTE).
FIGs. 3A and 3B show nitrogen adsorption (A) and pore size distribution (B) for BTE fabric modification.
FIGs. 4A and 4B show fluorescence characteristics (A) for the meso-tetra(4-carboxyphenyl) porphyrin (C4) functionalized DEB-BTE fabric sample and target adsorption (B) by the C4 DEB-BTE fabric.
FIGs. 5A and 5B show water vapor permeation through fabrics. (A) ACU fabric alone, with the DEB-BTE modification, and with the C4 porphyrin on the DEB-BTE modification. (B) Cotton fabric alone, with the BTE modification, with the CuDIX porphyrin on the BTE modification, and with the C4 porphyrin on the DEB-BTE modification.
FIGs. 6A and B show methyl salicylate permeation through fabrics. (A) Cotton fabric alone, with the DEB-BTE modification, and with the C4 porphyrin on the DEB-BTE modification. (B) ACU fabric alone, with the DEB-BTE modification, and with the C4 porphyrin on the DEB-BTE modification.
FIGs. 7A and 7B show paraoxon droplet permeation through cotton modified with C4 DEB-BTE. (A) target extracted from adsorbent swab and (B) target extracted from the modified cotton. Results from both illuminated and dark experiments are presented.
FIGs. 8A and 8B show shows CEES (A) and phosgene (B) permeation through fabrics. (A) Cotton fabric alone, with the BTE modification, and with the CuDIX porphyrin on the BTE modification. (B) ACU fabric CuDIX porphyrin on the BTE modification equilibrated at 0% and 45% relative humidity.

### DETAILED DESCRIPTION

### Definitions

Before describing the present invention in detail, it is to be understood that the terminology used in the specification is for the purpose of describing particular embodiments, and is not necessarily intended to be limiting. Although many methods, structures and materials similar, modified, or equivalent to those described herein can be used in the practice of the present invention without undue experimentation, the preferred methods, structures and materials are described herein. In describing and claiming the present invention, the following terminology will be used in accordance with the definitions set out below.

As used in this specification and the appended claims, the singular forms "a", "an," and "the" do not preclude plural referents, unless the content clearly dictates otherwise.

As used herein, the term "and/or" includes any and all combinations of one or more of the associated listed items.

As used herein, the term "about" when used in conjunction with a stated numerical value or range denotes somewhat more or somewhat less than the stated value or range, to within a range of ±10% of that stated.

### Description

The fabric modifications described here provide the potential for designing fabrics that provide a barrier to penetration of threat agents. They can be tailored to provide varied selectivity and binding capacity for different targets. Modifications can be made to a range of different types of fabrics facilitating application to individual and collective protection scenarios. The sorbents can be further modified using catalytic, antimicrobial, or other functional groups. This capability provides the opportunity for development of self-decontaminating materials or materials for utilization in sensing applications.

Described herein are modification of fabrics using a microwave initiation technique to produce a porous coating on the fibers providing adsorbent properties as well as the potential for further modification by functional groups (*e.g*., porphyrins), catalysts, and optical indicators.

Periodic mesoporous organosilicas (PMOs) are organic-inorganic materials with highly ordered pore networks and large internal surface areas (typically > 1,000 m²/g). The materials are synthesized using a surfactant template approach. *See* refs. 1-3. and have narrow pore size distributions with few blocked pores or obstructions facilitating molecular diffusion throughout the pore networks. The alternating siloxane and organic moieties give PMOs properties associated with both organic and inorganic materials. *See* refs. 4, 5. The siloxane groups provide the structural rigidity required to employ surfactant templating methods which provide precise control when engineering porosity. The incorporated organic groups provide binding characteristics which are normally associated with organic polymers. It is also possible to synthesize materials with morphological properties spanning several length scales. These hierarchical structures can be used to provide improved accessibility to the surface area of the materials.

PMOs have been applied to adsorption of targets from aqueous solution as well as to the adsorption of vapors. *See* refs.6-8. They are considered exceptionally suitable for catalytic applications. The well-organized pore systems and large pore diameters provide an avenue for incorporation of complex structures that are not feasible in amorphous aero- and xerogels. In addition, the generation of higher molecular weight products can be accommodated. *See* refs. 9-11. Efforts have resulted in the development of a range of PMO and hierarchical materials providing binding affinity and capacity for targets such as nitroenergetics, phosgene, ammonia, and organophosphates. *See* refs. 12-15. The incorporation of porphyrins into PMOs provides a high density of binding sites with specificity for the targeted contaminant and brings the catalyst/indicator and target into close proximity. Both the PMO and porphyrin components of the materials are highly stable resisting extremes in temperature and offering extended shelf lives with no need for special storage conditions. These materials have been applied to adsorption, detection, and photocatalytic conversion of targets. *See* refs. 16-24.

Previously described PMO materials have been applied in powder or thin film formats. Described herein is a method for application of similar techniques to the generation of functionalized fabrics in order to bring the capabilities of the organosilicate and porphyrin-modified organosilicate sorbents to protective applications. The envisioned applications include protective garments, shelters, and filtration materials.

### Description and Operation

### Microwave initiation

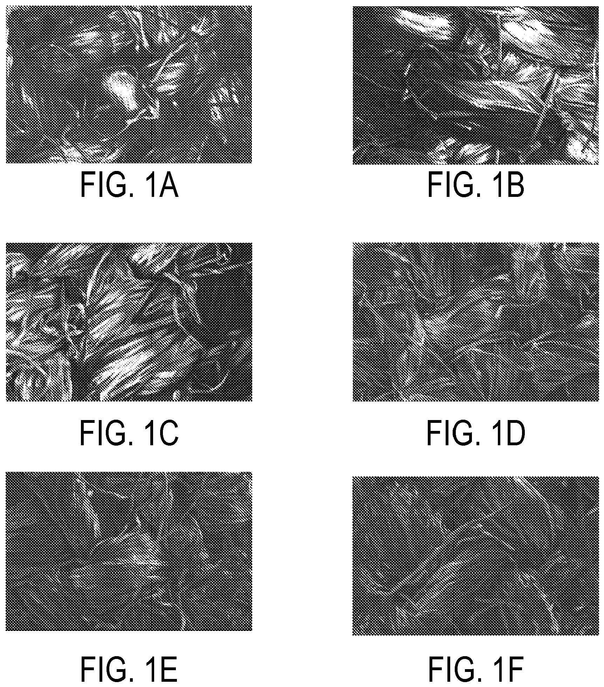

Fabric samples including unbleached cotton and military uniform fabrics (the Army Combat Uniform and Multi-CAM) were modified using tetraethylorthosilicate (TEOS). The protocol was developed based on a technique used for creating hydrophobic coatings on fabrics (*see* ref. 25), however, instead of making a hydrophobic coating, the chemistry used in this process provides sites that can be further modified through silane chemistry allowing for a subsequent dip coating process. SEM images of the three starting fabrics are shown in FIGs. 1A - 1C. Both the ACU (FIG. 1B) and multi-cam fabrics (FIG. 1C) have flat fibers. Those of the ACU are more smoothly woven and have less texture on the surface of the fibers. The Multi-cam fabrics have fibers that are slightly smaller than those of the ACU. The fibers of the cotton fabric (FIG. 1A), while still flattened in one dimension are thicker and narrower than those of the other fabrics. The cotton fabric has the loosest weave of the three fabrics

The microwave initiated technique involves wetting the fabric in a solution containing 1% ammonium hydroxide and 3% silane precursor in isopropyl alcohol. The excess liquid is gently squeezed from the fabric and it is immediately microwaved (1000 W) for 30 sec. The sample is then moved to an oven at 110°C and dried. The wetting/microwave process can be repeated multiple times for increased coverage. TEOS precursor was used to prepare 1 cycle, 3 cycle, and 10 cycle samples. Control samples using only the base catalyst with the rest of the process were prepared for comparison. SEM images for the materials are in FIGs. ID - 1F. Changes in the surface of the fibers are obvious as the number of application cycles is increased. A significant increase in roughness was observed for the 10-cycle as compared to the 1- or 3-cycle additions

### Dip Coating

Two materials previously developed for synthesis in monolithic formats were adapted for use in fabric modification. *See* refs. 15, 21, 26, 27. The first material utilizes a mixture of ethane and diethylbenzene bridging groups to provide binding affinity and capacity for organophosphate targets. The dip solution was prepared by mixing 3.5 g Pluronic F127, 2.62 g 1,2-bis(trimethoxysilyl)ethane (BTE), 1.56 g 1,4-bis(trimethoxysilylethyl)benzene (DEB), and 6 g methanol at room temperature. After thoroughly mixing, 1.5 g 0.05 M hydrochloric acid was added drop-wise. The solution was stirred for a minimum of 5 h up to 24 h with a tightly sealed lid. Fabric swatches were dipped into the preparation at 150 mm/min insuring that the wet fabric did not come into contact with solid surfaces. The swatches were cured while hanging at 60°C for 24 h followed by 80°C for 24 h. The Pluronic surfactant was then removed by heating the material in ethanol at 65°C for 48 h followed by air drying at room temperature.

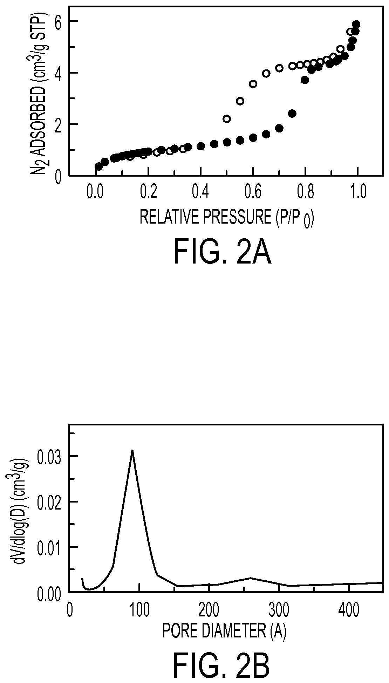

The resulting cotton samples consisted of approximately 2.1 mg of sorbent per square centimeter of fabric surface. Overall, samples had a BET surface area of 3.6 m²/g and a pore volume of 0.01 cm3/g with an average pore diameter of 90Å (FIG. 2B). When these values are corrected for the weight of the fabric which has no significant inherent surface area or pore volume, the sorbent has a surface area of 22 m²/g and a pore volume of 0.06 cm³/g. FIGs. 2A and 2B show nitrogen adsorption (A) and pore size distribution (B) for DEB-BTE fabric modification.

The second adapted material utilized only ethane bridging groups to provide a scaffold for further modification. The dip solution was prepared by mixing 1.9 g Pluronic P123, 0.5 g mesitylene, 2.12 g BTE, and 2 g methanol at room temperature. After thoroughly mixing, 6.07 g 0.1 M nitric acid was added drop-wise. The solution was stirred for 6 h with a tightly sealed lid. Fabric swatches were dipped into the preparation at 150 mm/min insuring that the wet fabric did not come into contact with solid surfaces. The swatches were cured while hanging at 60°C for 24 h followed by 80°C for 24 h. The Pluronic surfactant was then removed by heating the material in ethanol at 65°C for 48 h followed by air drying at room temperature. Fabric swatches were dipped into the preparation at 150 mm/min while ensuring that the wet fabric did not come into contact with solid surfaces. The swatches were cured while hanging at 60°C for 24 h followed by 80°C for 24 h. The Pluronic surfactant was then removed by heating the material in ethanol at 65°C for 48 h followed by air drying at room temperature

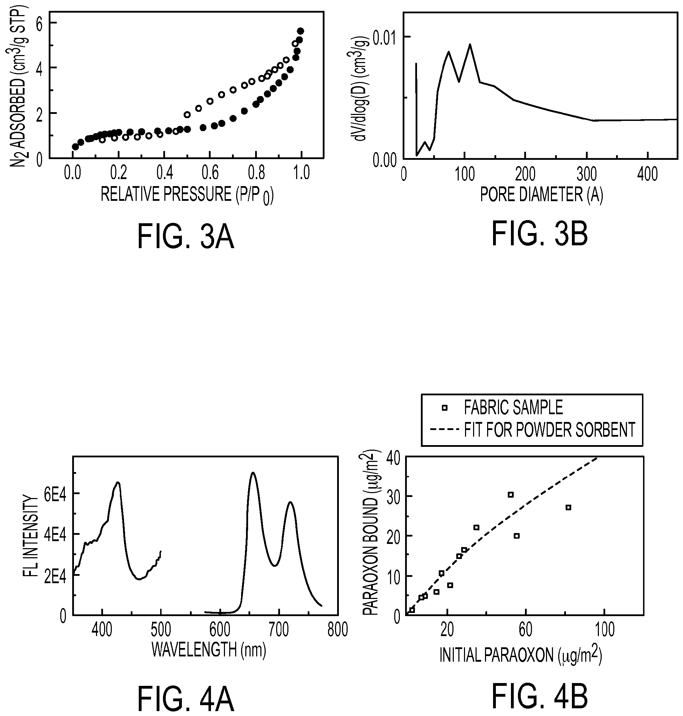

Many of the applications developed around organosilicate sorbents are based on post-synthesis modification. In order to compare the modified fabrics to the powdered materials, the fabrics were similarly modified. Primary amine groups were added to the sorbent surfaces by incubating the modified fabrics in a solution of 3-aminopropyltriethoxysilane (APS) in toluene for 1 h. They were then rinsed in toluene and dried at 110°C overnight. Porphyrin incorporation into the amine-functionalized sorbent was accomplished using 1-ethyl-3-(3-dimethylaminopropyl)carbodiimide (EDC) coupling chemistry. The material was placed in a solution of 5 mM EDC and 0.6 mM porphyrin in 100 mM MES buffer (2-(N-morpholino)ethanesulfonic acid). The solution was incubated overnight with agitation. The material was then rinsed with ethanol and water. Metals were incorporated into the porphyrin-functionalized material by heating in a solution of 1 mM metal salt in deionized water overnight. FIGs. 3A and 3B show nitrogen adsorption (A) and pore size distribution (B) for BTE fabric modification.

### Target adsorption, permeation, and photocatalysis

The fluorescence characteristics of fabric modified as above with meso-tetra(4-carboxyphenyl) porphyrin (C4) were evaluated. The characteristics were as expected, with slight indications of stacking as noted for the powdered materials. The characteristic excitation and emission bands were observed. Adsorption of targets from solution was also evaluated. The amount of adsorbed target was compared to the fit obtained for binding of paraoxon by the powdered materials. As shown in FIGs. 4A and 4B, the fabrics perform comparably to the powdered materials. FIGs. 4A and 4B show fluorescence characteristics (A) for the C4 functionalized DEB-BTE fabric sample and adsorption of the target paraoxon (B) by the same C4 DEB-BTE fabric.

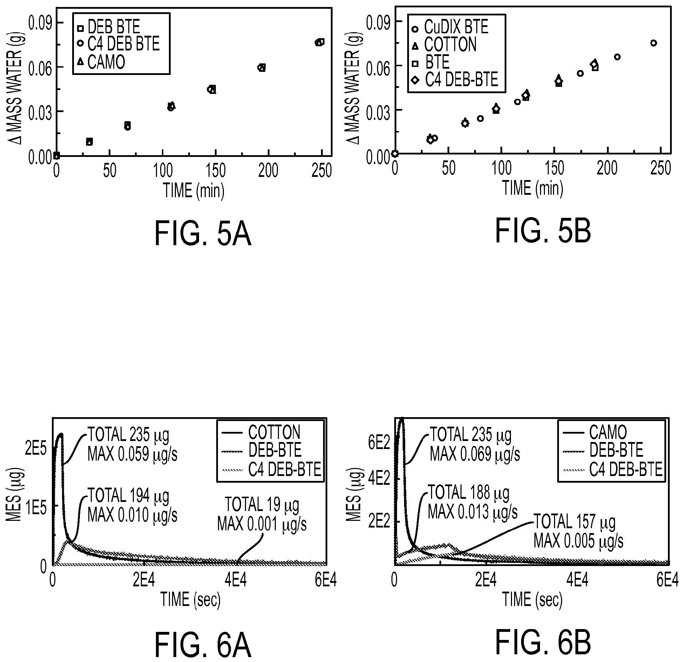

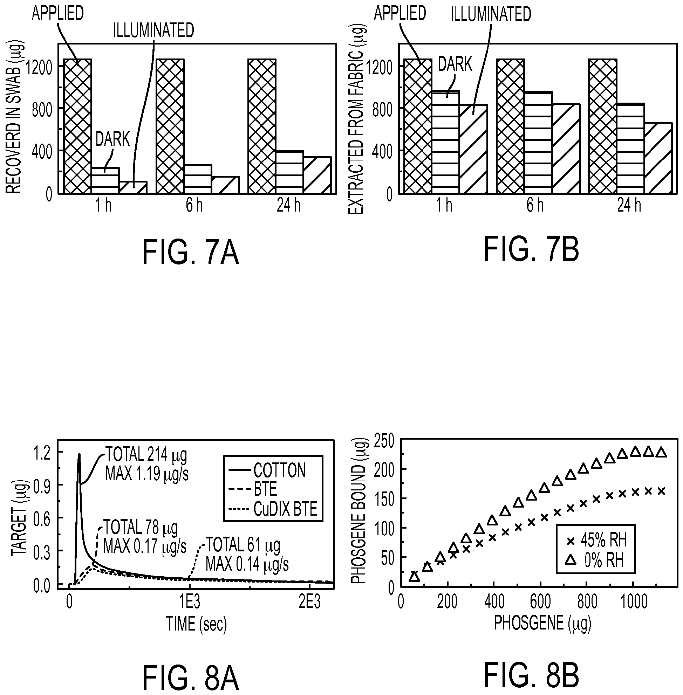

For application to a protective garment, water permeation is a serious consideration. The transport of water across the material is necessary for comfort as well as for thermal regulation. When the functionalized materials were evaluated, water transport across the functionalized fabrics was found to be nearly identical to that across the base fabrics (FIGs. 5A and 5B). Permeation of targets, on the other hand, was strongly impacted by functionalization of the fabrics. Here, methyl salicylate was used as a model compound. FIGs. 6A and B present data for both the cotton and ACU fabrics with/without the DEB-BTE modification and with/without C4 porphyrin. Both the total target passing through the material (area under the curve) and the peak target (maximum concentration) were reduced for the functionalized materials with the porphyrin functionalization showing the greatest reduction in target permeation. The BTE modification with copper Deuteroporphyin IX (CuDIX) is designed for the capture of targets such as phosgene and HD mustard. FIGs. 7A and 7B show permeation of half mustard (2-chloroethyl ethyl sulfide; CEES) and phosgene through the materials. In the CEES evaluation a single layer of fabric is used while the phosgene analysis utilized pleated material with a total of 12 layers. Both evaluations demonstrate significant target removal by the functionalized fabrics.

Droplet permeation through fabrics is also a consideration for protective applications. Targets delivered as aerosols may form droplets on tent fabrics for which there is no pressure applied. For a protective garment, droplets may be acquired through leaning or pressing a part of the body against a previously contaminated surface, for example kneeling on contaminated flooring. The C4 DEB-BTE materials were evaluated to determine how they would perform under this type of situation. The materials were evaluated under both dark and illuminated conditions to assess the potential of photocatalytic activity for removal of the target. Fabric samples (cotton) were placed on top of an adsorbent swab and a droplet of paraoxon was placed on the fabric. In the case of the cotton fabric, 75% of the paraoxon passed through the fabric and into the swab within the first hour. The remainder of the target was adsorbed into the fibers of the cotton and could be extracted. For the functionalized fabric (FIGs. 8A and 8B), approximately 20% of the target passed through the sample with no illumination after 1 h with a total of 32% on the swab after 24 h. Under illumination, 7% passed through the fabric to the swab in the first hour with a total of 26% after 24 h. In the absence of illumination, target not recovered from extraction of the swab was recovered when the fabric was extracted. Under illumination the total target recovered from the swab and the fabric was less than that applied indicating photocatalytic activity in the modified fabric.

Materials of the type described here provide the potential for increased reactive or catalytic surface area in protective applications. They also provide the potential for selectivity in the design of protective fabrics. These approaches can be applied to the generation of garments, shelters, or pleated filtration materials.

### Concluding Remarks

Although the present invention has been described in connection with preferred embodiments thereof, it will be appreciated by those skilled in the art that additions, deletions, modifications, and substitutions not specifically described may be made without departing from the scope of the invention. Terminology used herein should not be construed as being "means-plus-function" language unless the term "means" is expressly used in association therewith.

### References

1. C. T. Kresge; M. E. Leonowicz; W. J. Roth; J. C. Vartuli; J. S. Beck, 1992, "Ordered mesoporous molecular sieves synthesized by a liquid-crystal template mechanism," Nature 359, 710-712.
2. M. C. Burleigh; M. A. Markowitz; E. M. Wong; J. S. Lin; B. P. Gaber, 2001, "Synthesis of periodic mesoporous organosilicas with block copolymer templates," Chem Mater 13, 4411-4412.
3. Y. Goto; S. Inagaki, 2002, "Synthesis of large-pore phenylene-bridged mesoporous organosilica using triblock copolymer surfactant," Chem Commun 2410-2411.
4. R. J. P. Corriu; D. Leclercq, 1996, "Recent developments of molecular chemistry for sol-gel processes," Angewandte Chemie-International Edition in English 35, 1420-1436.
5. Q. S. Huo; D. I. Margolese; G. D. Stucky, 1996, "Surfactant control of phases in the synthesis of mesoporous silica-based materials," Chem Mater 8, 1147-1160.
6. K. Kosuge; T. Murakami; N. Kikukawa; M. Takemori, 2003, "Direct Synthesis of Porous Pure and Thiol-Functional Silica Spheres through the S+X-I+ Assembly Pathway," Chem. Mater. 15, 3184-9.
7. A. Matsumoto; H. Misran; K. Tsutsumi, 2004, "Adsorption characteristics of organosilica based mesoporous materials," Langmuir 20, 7139-7145.
8. A. Palaniappan; X. Su; F. E. H. Tay, 2006, "Functionalized mesoporous silica films for gas sensing applications," J Electroceram 16, 503-505.
9. F. Hoffmann; M. Cornelius; J. Morell; M. Fröba, 2006, "Silica-Based Mesoporous Organic-Inorganic Hybrid Materials," Angew Chem Int Edit 45, 3216-3251.
10. J. Weitkamp; M. Hunger; U. Rymsa, 2001, "Base catalysis on microporous and mesoporous materials: recent progress and perspectives," Micropor Mesopor Mat 48, 255-270.
11. D. Brunel; F. Fajula; J. B. Nagy; B. Deroide; M. J. Verhoef; L. Veum; J. A. Peters; H. van Bekkum, 2001, "Comparison of two MCM-41 grafted TEMPO catalysts in selective alcohol oxidation," Applied Catalysis a-General 213, 73-82.
12. B. J. Johnson; B. J. Melde; P. T. Charles; D. C. Cardona; M. A. Dinderman; A. P. Malanoski; S. B. Qadri, 2008, "Imprinted Nanoporous Organosilicas for Selective Adsorption of Nitroenergetic Targets," Langmuir 24, 9024-9029.
13. B. J. Johnson; B. J. Melde; P. T. Charles; M. A. Dinderman; A. P. Malanoski; I. A. Leska; S. A. Qadri, 2010, "Macroporous Silica for Concentration of Nitroenergetic Targets," Talanta 81, 1454-60.
14. B. J. Johnson; B. J. Melde; I. A. Leska; P. T. Charles; A. D. Hewitt, 2011, "Solid-phase extraction using hierarchical organosilicates for enhanced detection of nitroenergetic targets," J Environ Monitor 13, 1404-9.
15. B. J. Johnson; B. J. Melde; G. W. Peterson; B. J. Schindler; P. Jones, 2012, "Functionalized organosilicate materials for irritant gas removal," Chem Eng Sci 68, 376-382.
16. B. J. Johnson; N. E. Anderson; P. T. Charles; A. P. Malanoski; B. J. Melde; M. Nasir; J. R. Deschamps, 2011, "Porphyrin-Embedded Silicate Materials for Detection of Hydrocarbon Solvents," Sensors 11, 886-904.
17. B. J. Johnson; I. A. Leska; B. J. Melde; J. R. Taft, 2012, "Removal of phosgene by metalloporphyrin-functionalized porous organosilicates " Catal. Commun. 27, 105-8.
18. B. J. Johnson; I. A. Leska; B. J. Melde; J. R. Taft, 2012, "Self-Reporting Materials: Dual Use for Porphyrin-Embedded Sorbents," Sensor Actuat B-Chem 176, 399-404.
19. B. J. Johnson; B. J. Melde; P. T. Charles; A. P. Malanoski, 2009, "Porphyrin-embedded organosilicas for detection and decontamination," 2009 SPIE International Defense, Security and Sensing Symposium, Orlando, FL, #14.
20. B. J. Johnson; B. J. Melde; B. Lin; P. T. Charles; A. P. Malanoski; M. Nasir, 2010, "Functional and functionalized silicate materials," 2010 MRS Fall Meeting, Boston.
21. B. J. Johnson; B. J. Melde; C. Thomas; A. P. Malanoski; I. A. Leska; P. T. Charles; D. A. Parrish; J. R. Deschamps, 2010, "Fluorescent Silicate Materials for the Detection of Paraoxon," Sensors 10, 2315-2331.
22. B. J. Johnson; G. W. Peterson; P. Jones; B. J. Melde; J. R. Taft; B. J. Schindler, 2012, "Porphyrin-embedded organosilicate materials for ammonia adsorption," J Porphyr Phthalocya 16, 1-10.
23. B. Johnson-White; M. Zeinali; A. P. Malanoski; M. Dinderman, 2007, "Sunlight catalyzed conversion of cyclic organics with novel mesoporous organosilicas," Catalysis Comm 8, 1052-1056.
24. B. Johnson-White; M. Zeinali; K. M. Shaffer; J. C. H. Patterson; P. T. Charles; M. A. Markowitz, 2007, "Detection of organics using porphyrin embedded nanoporous organosilicas," Biosens Bioelectron 22, 1154-1162.
25. R. A. Hayn; J. R. Owens; S. A. Boyer; R. S. McDonald; H. J. Lee, 2011, "Preparation of highly hydrophobic and oleophobic textile surfaces using microwave-promoted silane coupling," Journal of Materials Science 46, 2503-2509.
26. K. Nakanishi; T. Amatani; S. Yano; T. Kodaria, 2008, "Multiscale Templating of Siloxane Gels via Polymerization-Induced Phase Separation," Chem Mater 20, 1108-1115.
27. K. Nakanishi; Y. Kobayashi; T. Amatani; K. Hirao; T. Kodaira, 2004, "Spontaneous Formation of Hierarchical Macro-Mesoporous Ethane-Silica Monolith," Chem Mater 16, 3652-3658.

### Related Patent

Norton, F.J., Schenectady, N.Y. "Waterproofing treatment of materials" US 2,386,259 October 1945.

### Related Publications

J.R. Owens; R.A. Hayn; S.A. Boyer; R.S. McDonald, 2008, "Synthesis of hydrophobic and oleophobic nylon-cotton fabric through microwave catalyzed silane attachment," AFRL Report #AFRL-RX-TY-TP-2008-4619, available through DTIC.
R.A. Hayn; J.R. Owens; S.A. Boyer; R.S. McDonald; H.J. Lee, 2011, "Preparation of highly hydrophobic and oleophobic textile surfaces using microwave-promoted silane coupling," Journal of Materials Science 46, 2503-2509.
K. Nakanishi; T. Amatani; S. Yano; T. Kodaria, 2008, "Multiscale Templating of Siloxane Gels via Polymerization-Induced Phase Separation," Chem Mater 20, 1108-1115.
K. Nakanishi; Y. Kobayashi; T. Amatani; K. Hirao; T. Kodaira, 2004, "Spontaneous Formation of Hierarchical Macro-Mesoporous Ethane-Silica Monolith," Chem Mater 16, 3652-3658.
B. J. Johnson; B. J. Melde; G. W. Peterson; B. J. Schindler; P. Jones, 2012, "Functionalized organosilicate materials for irritant gas removal," Chem Eng Sci 68, 376-382.
B. J. Johnson; I. A. Leska; B. J. Melde; J. R. Taft, 2012, "Removal of phosgene by metalloporphyrin-functionalized porous organosilicates " Catal. Commun. 27, 105-8.
B. J. Johnson; I. A. Leska; B. J. Melde; J. R. Taft, 2012, "Self-Reporting Materials: Dual Use for Porphyrin-Embedded Sorbents," Sensor Actuat B-Chem 176, 399-404.
B. J. Johnson; B. J. Melde; C. Thomas; A. P. Malanoski; I. A. Leska; P. T. Charles; D. A. Parrish; J. R. Deschamps, 2010, "Fluorescent Silicate Materials for the Detection of Paraoxon," Sensors 10, 2315-2331.
S. Sundarrajan, A.R. Chandrasekaran, S. Ramakrishna "An Update on Nanomaterials-Based Textiles for Protection and Decontamination," J. Am. Ceram. Soc. 2010, 93, 3955-3975.

## Claims

1. A method of treating fabric, the method comprising:
wetting a fabric in a first solution comprising tetraethylorthosilicate (TEOS) to obtain a precursor fabric,
irradiating the precursor fabric with microwave radiation to obtain a TEOS functionalized fabric,
dip-coating the TEOS functionalized fabric in a dip solution comprising surfactant and organosilica precursor to obtain dipped fabric, and
curing the dipped fabric to obtain a modified fabric,
wherein said organosilica precursor comprises:
(1) bis(trimethoxysilyl)ethane (BTE) and 1,4-bis(trimethoxysilylethyl)benzene (DEB); or
(2) mesitylene and BTE.

2. The method of Claim 1, wherein said first solution further comprises ammonium hydroxide.

3. The method of Claim 1, wherein said organosilica precursor comprises bis(trimethoxysilyl)ethane (BTE), the method further comprising chemically coupling a porphyrin to the BTE to obtain a porphyrin-functionalized fabric.

4. The method of Claim 3, wherein said porphyrin is selected from the group consisting of meso-tetra(4-carboxyphenyl) porphyrin and copper Deuteroporphyin IX.

5. The method of Claim 1, further comprising modifying the fabric with a functional group, catalyst, or optical indicator.

6. A modified fabric comprising:
a fabric modified by wetting the fabric in a first solution comprising tetraethylorthosilicate (TEOS) to obtain a precursor fabric, irradiating the precursor fabric with microwave radiation to obtain a TEOS functionalized fabric, dip-coating the TEOS functionalized fabric in a dip solution comprising surfactant and organosilica precursor to obtain dipped fabric, and curing the dipped fabric to obtain the modified fabric.

7. The modified fabric of Claim 6, wherein a porphyrin is chemically coupled to the fabric.

8. The modified fabric of Claim 6, wherein a functional group, catalyst, or optical indicator is chemically coupled to the fabric.

9. A garment, a shelter, or a filter comprising the modified fabric of Claim 6.

## Patentansprüche

1. Ein Verfahren zur Behandlung von Geweben, wobei das Verfahren Folgendes umfasst:
Benetzen eines Gewebes in einer ersten Lösung, die Tetraethylorthosilicat (TEOS) enthält, um ein Ausgangsgewebe zu erhalten,
Bestrahlen des Ausgangsgewebes mit Mikrowellenstrahlung, um ein TEOS-funktionalisiertes Gewebe zu erhalten,
Tauchbeschichten des TEOS-funktionalisierten Gewebes in einer Tauchlösung, die ein Tensid und einen Organosilica-Vorläufer enthält, um ein getauchtes Gewebe zu erhalten, und
Nachbehandeln des getauchten Gewebes, um ein modifiziertes Gewebe zu erhalten,
wobei der Organosilica-Vorläufer Folgendes enthält:
(1) Bis(trimethoxysilyl)ethan (BTE) und 1,4-Bis(trimethoxysilylethyl)benzol (DEB), oder
(2) Mesitylen und BTE.

2. Verfahren nach Anspruch 1, wobei die erste Lösung ferner Ammoniumhydroxid enthält.

3. Verfahren nach Anspruch 1, wobei der Organosilica-Vorläufer Bis(trimethoxysilyl)ethan (BTE) enthält, wobei das Verfahren ferner die chemische Kopplung eines Porphyrins an das BTE umfasst, um ein porphyrinfunktionalisiertes Gewebe zu erhalten.

4. Verfahren nach Anspruch 3, wobei das Porphyrin ausgewählt ist aus der Gruppe bestehend aus meso-Tetra(4-Carboxyphenyl)porphyrin und Kupfer-Deuteroporphyrin IX.

5. Verfahren nach Anspruch 1, ferner umfassend das Modifizieren des Gewebes mit einer funktionellen Gruppe, einem Katalysator oder einem optischen Indikator.

6. Modifiziertes Gewebe, umfassend:
ein Gewebe, modifiziert durch Benetzen des Gewebes in einer ersten Lösung, die Tetraethylorthosilicat (TEOS) enthält, um ein Ausgangsgewebe zu erhalten, Bestrahlen des Ausgangsgewebes mit Mikrowellenstrahlung, um ein TEOS-funktionalisiertes Gewebe zu erhalten, Tauchbeschichten des TEOS-funktionalisierten Gewebes in einer Tauchlösung, die ein Tensid und einen Organosilica-Vorläufer enthält, um ein getauchtes Gewebe zu erhalten, und Nachbehandeln des getauchten Gewebes, um das modifizierte Gewebe zu erhalten.

7. Modifiziertes Gewebe nach Anspruch 6, wobei ein Porphyrin chemisch mit dem Gewebe gekoppelt ist.

8. Modifiziertes Gewebe nach Anspruch 6, wobei eine funktionelle Gruppe, ein Katalysator oder ein optischer Indikator chemisch mit dem Gewebe gekoppelt ist.

9. Ein Kleidungsstück, eine Schutzhülle oder ein Filter, umfassend das modifizierte Gewebe nach Anspruch 6.

## Revendications

1. Procédé de traitement de tissu, le procédé comprenant :
le mouillage d'un tissu dans une première solution comprenant de l'orthosilicate de tétraéthyle (TEOS) afin d'obtenir un tissu précurseur,
l'irradiation du tissu précurseur avec un rayonnement micro-ondes pour obtenir un tissu fonctionnalisé par le TEOS,
l'enduction par trempage du tissu fonctionnalisé par le TEOS dans une solution de trempage comprenant un tensioactif et un précurseur d'organosilice afin d'obtenir un tissu trempé, et
le durcissement du tissu trempé afin d'obtenir un tissu modifié,
ledit précurseur d'organosilice comprenant :
(1) du bis(triméthoxysilyl)éthane (BTE) et du l,4-bis(triméthoxysilyléthyl)benzène (DEB) ; ou
(2) du mésitylène et du BTE.

2. Procédé selon la revendication 1, dans lequel ladite première solution comprend en outre de l'hydroxyde d'ammonium.

3. Procédé selon la revendication 1, dans lequel ledit précurseur d'organosilice comprend du bis(triméthoxysilyl)éthane (BTE), le procédé comprenant en outre le couplage chimique d'une porphyrine au BTE afin d'obtenir un tissu fonctionnalisé par de la porphyrine.

4. Procédé selon la revendication 3, dans lequel ladite porphyrine est sélectionnée dans le groupe constitué de méso-tétra(4-carboxyphényl)porphyrine et de coprodeutéroporphyrine IX.

5. Procédé selon la revendication 1, comprenant en outre la modification du tissu avec un groupe fonctionnel, un catalyseur ou un indicateur optique.

6. Tissu modifié comprenant :
un tissu modifié par mouillage du tissu dans une première solution comprenant de l'orthosilicate de tétraéthyle (TEOS) afin d'obtenir un tissu précurseur, l'irradiation du tissu précurseur avec un rayonnement micro-ondes afin d'obtenir un tissu fonctionnalisé par le TEOS, l'enduction par trempage du tissu fonctionnalisé par le TEOS dans une solution de trempage comprenant un tensioactif et un précurseur d'organosilice afin d'obtenir un tissu trempé et le durcissement du tissu trempé afin d'obtenir le tissu modifié.

7. Tissu modifié selon la revendication 6, une porphyrine étant couplée chimiquement au tissu.

8. Tissu modifié selon la revendication 6, un groupe fonctionnel, un catalyseur ou un indicateur optique étant couplé chimiquement au tissu.

9. Vêtement, abri ou filtre comprenant le tissu modifié selon la revendication 6.
